# EUROPEAN PATENT SPECIFICATION

(11) **EP 3 454 978 B1**
(45) Date of publication and mention of the grant of the patent: **29.12.2021**
(21) Application number: 17771891.3
(22) Date of filing: 11.05.2017
(51) Int. Cl.: B01D 71/02, C12N 15/10

(54) **GENOMIC DNA ISOLATION METHOD**
VERFAHREN ZUR ISOLIERUNG VON GENOMISCHER DNA
PROCÉDÉ D'ISOLEMENT D'ADN GÉNOMIQUE

(30) Priority: 12.05.2016 TR 201606310
(43) Date of publication of application: 20.03.2019
(73) Proprietor: Akdeniz Universitesi, Bulvari 07058 Kampus Antalya (TR)
(72) Inventor: KARACA, Mehmet, Tarla Bitkileri Bolumu 07059 Antalya (TR); INCE, Ayse Gul, Tarla Bitkileri Bolumu 07059 Antalya (TR)
(74) Representative: Sevinç, Cenk
(86) International application number: PCT/TR2017/050185
(87) International publication number: WO 2017/196285

(56) References cited:
- WO-A1-2011/104027
- JP-A- 2004 290 030
- Dneasy Plant ET AL: "Plant Handbook Sample & Assay Technologies", , 1 June 2015 (2015-06-01), XP055414757, Retrieved from the Internet: URL:https://www.qiagen.com/nl/resources/do wnload.aspx?id=95dec8a9-ec37-4457-8884-5de dd8ba9448&lang=en [retrieved on 2017-10-11]

## Description

### Technical Field

The invention relates to a genomic DNA isolation method, kit and production method thereof, that is economic, high quality and efficient, free of carcinogenic and mutagenic agents, with no negative impact on the environment and the researcher (applicator) in terms of health, and that can be used effectively on generative and vegetative plant parts and many plant varieties and species.

### State of The Art

DNA is a molecule that generally present a right handed helix structure that occur as two parallel chains formed by phosphoric acid and deoxyribose units encoded in the sequence of the nucleotides where it carries the genetic information nucleotide are cross-linked by the purine and pyrimidine bases. The prerequisite at the molecular biology and epigenetic techniques practices is to realize high-quality DNA isolation of adequate quantity from different gender, species, tissues or cells without damaging nucleotides and inducing any modifications. DNA in high-quality and quantity must be achieved before conducting the operations such as polymerase chain reaction (PZR) studies, DNA sequencing studies (DNA sequence analyses), DNA amplification, hybridization and next generation DNA sequencing that involve molecular biology practices.

DNA isolation basically comprises of 2 phases as cell wall fractionation and dissolution of DNA-protein complex and separation from other molecules, including the RNA. The cell wall must be fractionized in order to reach the genomic DNA at the nucleus of the cell. Fractionation might be achieved through physical and chemical means. Physically heated cells are also subjected to chemical mixtures in order to ensure wall fractionation. The salt available within said chemical mixture permeate through the cell wall and separate the proteins from the DNA. The detergent that improve permeability of the cell wall enables release of the cell contents. Ethylene diamine tetra acetic acid (EDTA) attaches to the cofactors such as magnesium etc. and inhibit the DNase activity, thus allowing the DNA to remain stable. proteolytic enzymes, on the other hand, lyse the proteins in the cell content, and enable formation of homogenous mixture in terms of subsequent steps. As the RNAs within the cell can be stable in the form of single chain, those RNAs should also be removed, and RNase enzyme is used for this purpose. The chemicals used during fractionation phase and the reaction time might vary depending on the material you wish to extract the DNA. For instance, when extracting the DNA of some bacteria, a different enzyme called lysozyme can be used for cell wall having a different structure and the time can be prolonged. Likewise, the fractionation time spent for DNA isolation from the blood is less than the time spent for DNA isolation from the tissue.

The method of dissolving the DNA-protein complex and separation from other molecules generally predicates on dissolution and denaturation. However, the technological advancements today enabled use of spin columns that retain DNA. Phenol extraction process is frequently used in denaturation based chemical dissolution and the proteins and the DNA are separated and diverge from each other through use of phenol and chloroform and centrifugal power. In physical attachment where spin columns are used, on the other hand, the DNA adheres to matrix layer specifically placed inside the tubes by use of special solutions. As the molecules other than DNA fail to adhere to the matrix, they are removed from the medium at the same phase though centrifugal or gravitational force. The spin columns are again subjected to the process so-called "washing" in order to completely remove the non-DNA substances though use of certain solutions. The last phase is the "elution" phase where the solutions that remove the DNA from the matrix are used, and with this last phase, the DNA gets its final form.

There are numerous other methods and kits available for DNA isolation. In the prior art, the applications no. EP2258845, US6027945, and US8729252 discloses methods for eDNA isolation, while the applications no. CN105002159, WO0040697 and WO2015132216 mentions about the kits used for DNA isolation.

In addition, the cDNA synthesized using the mRNA is another DNA source. In some cases, DNA should be isolated from PZR or hybridization studies. Regardless of the source, the DNA molecules are polymers resistant to degradation, and can be preserved for long time under suitable storage conditions and can be used at analyses recurrently. However, as the large fragments presents higher tendencies towards breaking and suffering damages compared to the small fragments, the DNA molecules intended to be isolated as large fragments should be processed very carefully. Isolation of large DNA fragments necessitate use of different methods.

The DNA to be purified can be genomic or non-genomic PZR or hybridization products as well as mitochondria (mtDNA) and chloroplast (chlDNA) DNA, or plasmid DNA from the bacteria. The increasing number and diversity of tests has brought up the necessity of automatic DNA isolation at the molecular diagnosis laboratories and automated instruments has been developed for this purpose. DNA isolation kits are standardized packages that allow us to isolate the genomic and non-genomic DNA molecules. There are DNA isolation kits available today that are designed for various purposes. Some types of isolation kits allow us to separate the desired DNA from others from agarose gels after PZR. This type of isolation kits comprises of platforms such as membrane, column, capillary, etc. that enable bonding of the DNA. The DNA isolation kits not only shorten the phases of the tests, but also are tools that allow more effective and easy DNA isolation.

The tissue fractionation process at the automatic DNA isolation systems generally uses magnetic ball technology. If, however, such magnetic particles are not removed from the medium at the elution step, they might affect the PZR amplification reaction and might lead to unrealistic negative or positive PZR outcomes.

In the prior art, recently the DNA boding columns so-called as DNA cartridges formed by processing the silica particles at the nano levels through special filters, are being developed wherein the solid phase for DNA isolation is silica.

DNeasy Plant Kits in the state of art are intended for molecular biology applications. DNeasy Plant Kits provide a fast and easy way to purify DNA from plant and fungal tissue. Purified DNA is eluted in low-salt buffer or water, ready for use in downstream applications. DNA purified using DNeasy Plant Kits is up to 40 kb in size, with fragments of 20-25 kb predominating

The kits developed for DNA isolation are generally capable of isolating adequate quantity and quality of genomic DNA from limited number of plant species. These kits which generally have higher costs also require technical information and infrastructure. In addition, chemicals such as the beta mercapto ethanol, phenol, chloroform used at some genomic DNA isolation kits present the potential to have negative impact on human and environmental health.

Taking into consideration the disclosures provided hereinabove and the disadvantages experienced as a result of the studies, the genomic DNA isolation method of the invention that allows DNA isolation in high quality and quantity with no negative impact in economic and health terms has been achieved.

### The Problems that the Invention Aims to Solve

The objective of the invention is to develop genomic DNA isolation method that is economic, high quality and efficient, free of carcinogenic and mutagenic agents, with no negative impact on the environment and the researcher (applicator) in terms of health, and that can be used effectively on generative and vegetative plant parts and many plant varieties and species.

Another objective of the invention is to ensure that DNA isolation can be achieved at live plants as well as from lifeless plant parts including the dried plant accessories and wheat and rice (paddy without embryo).

Another objective of the invention is to ensure that DNA isolation can be carried out in shorter time compared to the kits used for DNA isolation, at lower costs and without requiring high cost infrastructure.

Another objective of the invention is to form the DNA bonding column and to ensure reformation from used DNA bonding column parts.

Another objective of the invention is to reduce the number of disposable substances and to offer the opportunity to reuse the plastic materials used for DNA bonding column and .

### Description of the Figures

Figure 1: DNA Bonding column
Figure 2: Agarose Gel Electrophoresis Analysis Image
Figure 3: Polymerase Chain Reaction Analysis Image

### Description of References in the Figures

The parts on the figures are enumerated, and their descriptions are provided hereunder:
- 1 :: DNA Bonding column

### Description of the Invention

The invention relates to a genomic DNA isolation method that is economic, high quality and efficient, free of carcinogenic and mutagenic agents, with no negative impact on the environment and the researcher (applicator) in terms of health, and that can be used effectively on generative and vegetative plant parts and many plant varieties and species.

For DNA isolation, first of all, the DNA bonding column (1) should be formed and the tissue lesion solution, DNA bonding solution and DNA washing and separation from silica solutions should be prepared.

### FORMING THE DNA BONDING COLUMN

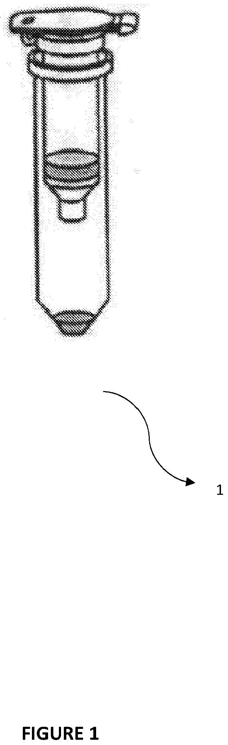

Three different types of DNA bonding columns (1) are used in the method of the invention developed from a multitude of live and lifeless plant species. The first column is the novel 1^{st} DNA bonding column (1), and is illustrated in Figure 1.

### 1^{st} DNA BONDING COLUMN

The chemicals required for column equilibrium solution (CES) used for the purpose of promoting higher quantity and quality of DNA molecules to bond to silica and ensure longer term availability of the columns for the 1^{st} DNA bonding column (1) are 0,2-1,2 M 3-morpholino propane-1-sulfonic acid (MOPS, pH 6,5-7,5), 2,4-5,6 M sodium chloride (NaCl), stock Triton-X-100 and Stock isopropanol.

For example, in order to prepare 30 mL of CES, 17, 55-21, 55 mL distilled water, 3 mL MOPS, 4,5 mL NaCl, 0,45-0,65 mL Triton-X-100 and 2,5-6,5 mL isopropanol is added in order. 1^{st} DNA bonding column (1) comprises the process steps of;
o Placing the Filter papers within the tube using a sterile forceps and loading bar and adding 15-67 mg silica thereon,
∘ After the silica surface equalizes, pressing the O-ring on at least one filter paper using the loading bar,
∘ Adding 0,20-0,75 mL column equilibrium solution (CES) to the column,
∘ Keeping the mixture at 22-26°C for 1-3 hours, and
∘ Centrifuging the mixture for 1-2 minutes at the rate of 6000-12000xg.

### 2^{nd} DNA BONDING COLUMN

2^{nd} DNA bonding column (1) prepared for the DNA isolation method is obtained from full silica based DNA bonding column (1) used previously. Obtaining 2^{nd} DNA bonding column (1) from used DNA bonding column (1) comprises the process steps of;
- Sorting the used DNA bonding column (1) according to the type of components such as exterior tube, inner tube, silica, supporting layers and O-ring,
- Keeping the components within 0,01-0,7 N hydrochloric acid (HCl) at stoichiometric proportion of at least 3 times by volume (3/1) for 25-36 hours,
- Rinsing the components with distilled water for at least 3 times and autoclaving,
- Adding, consecutively for 2-5 times, 0,5-0,75 mL 0,01-0,7 N HCl to the columns,
- Centrifuging the mixture for 1-2 minutes at the rate of 6000-12000xg,
- Centrifuging the empty DNA bonding column (1) for 1-2 minutes at the rate of 6000-12000xg,
- Adding 0,5-0,75 mL CES to the columns and keeping the same at 22-26°C for 1-3 hours, and
- Centrifuging the mixture for 1-2 minutes at the rate of 6000-12000xg.

### 3^{rd} DNA BONDING COLUMN

The 3^{rd} DNA bonding column (1) prepared for the DNA isolation method, on the other hand, is for checking whether the DNA solutions and methodology employed are compatible at the commercially available DNA bonding columns (1). Obtained results indicated that the solutions and methodology are compatible with all kinds of silica based DNA bonding column (1).

### PREPARING THE TISSUE LESION SOLUTIONS

Tris(hydroxyl methyl)amino methane (Tris), ethylendiaminetetraacetic acid (EDTA), sodium dodecyl sulfate (SDS), sodium laurel sulfate (SLS), Sodium deoxycholate (SDC), polyvinyl polypyrrolidone (PVPP) and sodium azide (NaN₃) are used for preparing the tissue lesion solutions. The solution containing the specified chemicals is called M₁. As the chemicals SDS, SLS and SDC present anionic characteristics, solutions M_{2A} and M_{2B} were formed in order to inhibit the bonding indicators to the silica particles that compete with the DNA molecules in DNA bonding column (1). These solutions should be used according to the condition of the samples, such as plant species and freshness, dryness, polysaccharide and oil contents. As the proteins fractionize by being processed by the proteinase K, the cellular RNA molecules (rRNA, mRNA, tRNA and other types of RNA) are washed with RNase enzyme in company with the lesion solution.

*Preparation of Solution M₁ comprises the process steps of;*
- Mixing 1-3 M Tris (pH 7,5-8,5) and 0,5-1,5 M (pH 7,5-8,5) EDTA,
- Adding Tris-EDTA mixture to distilled water,
- Autoclaving and sterilizing the mixture,
- Adding 12,5-25,5 mL 10-15% SDS, 12,5-25,5 mL 10-15% SLS, 12,5-15,5 mL 5-10% SDC, 25-50 mL 5-10% PVPP, 2,5-5,5 mL Triton-X-100, 2,5-5,5 mL Tween-20 and 1,2-2,75 mL 4-8% NaN₃ to the mixture,
- Filtering and sterilizing the solution.

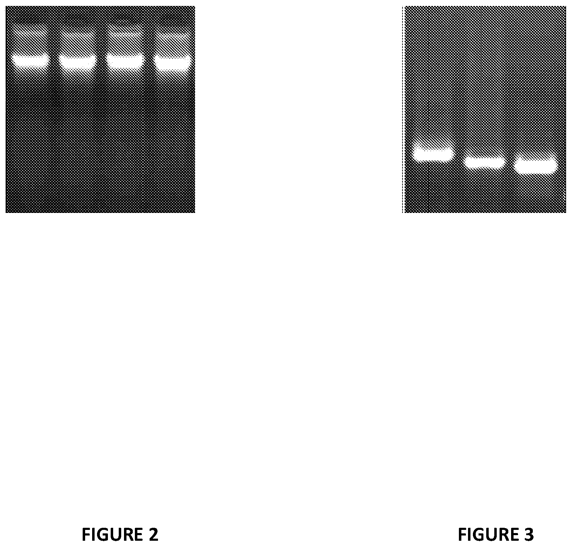

The RNase and Proteinase that are added to solution M₁ as exogen have been tested by employing spectrophotometric and electrophoretic (agarose gel) methods where K enzymes show activity. it is possible to see from the agarose gel electrophoreses analysis image shown in Figure 2 that there are no RNA residue and protein/polysaccharide residues. Moreover, it is further possible to see from the polymerase chain reaction analysis image shown in Figure 3 that the isolated samples lack proteinase K activity and that there is no residue that would prohibit DNA polymerase activity. In addition, Table 1 gives the spectrophotometer reading values for DNA samples obtained from various plant parts.

**Table 1. spectrophotometer reading values for DNA samples obtained from various plant parts**

| **Plant parts** | **A230** | **A260** | **A280** | **A260/A280** | **A260/A230** |
|---|---|---|---|---|---|
| **Root** | 0,126 | 0,225 | 0,123 | 1,79 | 1,83 |
| **Seed** | 0,213 | 0,417 | 0,225 | 1,96 | 1,85 |
| **Leave** | 0,120 | 0,228 | 0,123 | 1,90 | 1,85 |
| **Placenta** | 0,072 | 0,126 | 0,063 | 1,75 | 2,00 |
| **Petal** | 0,120 | 0,234 | 0,123 | 1,95 | 1,90 |
| **Gemma** | 0,225 | 0,384 | 0,210 | 1,71 | 1,83 |
| **Radicle** | 0,261 | 0,519 | 0,270 | 1,99 | 1,92 |
| **Pedicle** | 0,111 | 0,192 | 0,102 | 1,73 | 1,88 |
| **Embryo** | 0,108 | 0,219 | 0,117 | 2,03 | 1,87 |
| **Pollen** | 0,225 | 0,429 | 0,207 | 1,91 | 2,07 |

An A260/A280 ratio above 1,6 indicates that it is a usable DNA, and a ratio in the range of 1,7-2,1 indicates that it is a very high quality DNA. Moreover, if A260/A280 ratio is above 2,2, then some RNA molecules are present together with the DNA. An A260/A230 value in the range of 1,6-3,0 indicates that the DNA is of high quality, and if such ratio is above 3, then it indicates that some residues (some substances either isolated together with the DNA or added afterwards) affect absorbance. The spectrophotometric and agarose gel electrophoresis analyses clearly suggest that the RNase and Proteinase K enzymes added into the solution M₁ as exogen are active. When solution M₁ is used in conjunction with the exogen proteinase K and RNase enzymes, the proteins bonded to the cell wall and the cellular membranes and the DNA molecules are removed. RNase enzyme is used for the purpose of washing the RNA type nucleic acids thus preventing their isolation together with the DNA during DNA isolation. Moreover, the spectrophotometric analyses have confirmed that it doesn't involve microorganism activities at the room temperature for one year as it contains NaN₃.

*Preparation of Solution M_{2A} comprises the process steps of;*
- Dissolving 67,8-78,8 g potassium acetate (KCH₃COOH) in 60-80 mL distilled water,
- Adding 23-30 mL 12-14 M glacial acetic acid to the solution,
- Agitating the mixture,
- Adding 65-85 mL distilled water to the mixture.

*Preparation of Solution M_{2B} comprises the process steps of;*
- Mixing 42,8-56,2 g guanidine HCl and 30-42 mL 3,4-4,8 M NaCl,
- Adding distilled water until the volume of the solution reaches 100 mL.

### PREPARING DNA BONDING SOLUTIONS

The solutions that enable the DNA to bond to the silica column are called as M₃ and M₄, and some detergents, chaotropic agents and salts were used when preparing these solutions.

*Preparation of Solution* M₃ *comprises the process steps of,*
- Adding 50-80 mL M₂ solution to 105-115 g guanidine HCl,
- Adding 12,5-25,5 mL 80-100% Triton-X-100 and 67,5-87,5 mL distilled water to the mixture.

*Preparation of Solution M₄ comprises the process steps of,*
- Adding 165-195 mL 96-100% ethyl alcohol and 12,5-25,5 mL 80-100% Triton-X-100 to 37,8-54,3 g guanidine HCl,
- Adding ethyl alcohol to the mixture in order to increase the volume of the mixture to 200-250 mL.

### PREPARING DNA WASHING AND SEPARATION FROM SILICA SOLUTIONS

The solutions that enable the DNA to remain bonded to the silica particles and other molecules to detach from the silica particles are called as M₅ and M₆, and these solutions comprise of some detergents, chaotropic agent and alcohol. In addition, the solution denominated as M₇ is also used as DNA washing and separation from silica solution.

*Preparation of Solution M₅ comprises the process steps of,*
- Mixing 38,75-49,75 mL water with 25 mL 0,5-1,5 M MOPS (pH 7,2-7,9),
- Adding 50-75 mL 3,6-4,4 M NaCl and 1,25-2,50 mL 2-4% (w/v) NaN₃ to the mixture,
- Adding 25-50 mL M_{2A} solution and 50-100 mL 96-100% ethyl alcohol to the mixture.

*Preparation of Solution M₆ comprises the process steps of,*
- Mixing 8-12 mM Tris and 0,5-1,2 mM EDTA (pH 7,4-8,4),
- Adding 0,08-1,25 mL 5 M NaCl and 0,25-0,50 mL NaN₃ to the mixture,
- Adding 5,67-14,6 mL distilled water and 175-195 mL 96-100% ethyl alcohol to the mixture.

*Preparation of Solution M₇ comprises the process step of,*
- Mixing 25-35 mL 0,01-0,06 M Tris (pH 7,5-8,5) with 175-225 mL distilled water.

The DNA isolation process can be initiated upon preparation of the DNA solutions used . The DNA isolation process comprises of four phases, which are;
**A.** Tissue fractionation, expositing the cells and cell lesion,
**B.** Expositing the DNA molecules,
**C.** Bonding of the DNA molecules to the silica particles, and
**D.** Washing and separation of the DNA molecule from silica.

### A. Tissue fractionation, Expositing the cells and cell lesion;

The tissue fractionation process that varies based on the plant species and the type of the tissue differ for dry and fresh plant parts and plant seeds. Tissue fractionation, expositing the cells and cell lesion process comprises the process steps of;
- Fractionating the naturally dried or artificially dried plant parts, plant seeds and lyophilized samples at micro mills, and the fresh plant parts in liquid nitrogen,
- Collecting 0,03-0,08 g samples from dried plant parts, and 0,1-0,3 g samples from fresh tissue parts, and 0,1-0,15 g from plant seeds and placing the samples into 1,5-2,0 mL tubes,
- Adding 300-600 µL M₁ solution and 10-20 µL 20 mg/mL Proteinase K enzyme to the plant samples,
- Mixing the obtained solutions for 30-90 seconds,
- Keeping the mixtures at 50-70°C for 10-20 minutes and agitating the mixtures for 30-45 seconds once per every 5 minutes.

### B. Expositing the DNA molecules

The expositing the DNA molecules step realized after incubation at the final step of tissue fractionation and cell lesion process comprises the process steps of;
- Adding solution M_{2A} or M_{2B} to the mixtures at half the stoichiometric ratio (1/2) of solution M₁ by volume,
- Mixing the obtained solutions for 30-60 seconds,
- Keeping the mixtures at 50-70°C for 10-20 minutes and agitating the mixtures for 15-45 seconds once per every 5 minutes,
- Following incubation, centrifuging the solution at 24-26°C for 10-20 minutes at the rate of 6000-12000xg.

### C. Bonding of the DNA molecules to the silica particles

Bonding of the DNA molecules to the silica particles initiated by collecting 0.5 mL from centrifuged supernatant comprises the process steps of;
- Adding 0,25-0,5 mL solution M₃ and 0,25-0,5 mL ethanol or isopropanol to supernatant,
- Agitating the mixtures for 30-60 seconds,
- Transferring 0,5-0,7 mL to the DNA bonding column (1),
- Centrifuging the DNA bonding column (1) at 24-26°C for 1-2 minutes at the rate of 6000-11000xg and decanting the solution collected at the exterior tube,
- Repeating transfer of 0,5-0,7 mL solution to the DNA bonding column (1) and bonding of the DNA molecules to the silica particles,
- Adding 0,25-0,5 mL solution M₄ solution to the inner chamber of the DNA bonding column (1) for the purpose of selective bonding of the DNA molecules to the silica particles and removal of the pigments, and
- Centrifuging the mixture at 24-26°C for 1-2 minutes at the rate of 6000-11000xg and decanting the solution collected at the exterior tube.

### D. Washing the DNA molecule and separation of it from silica

The step of washing and separation of the DNA molecule from silica required as proteins, lipids, polysaccharide and pigment residues are linked to the DNA molecules and the silica surface comprises the process steps of;
- Adding 500-750 µL solution M₅ to the DNA bonding column (1),
- Centrifuging the mixture at 24-26°C for 1-2 minutes at the rate of 6000-11000xg and discharging the bottom chamber,
- Adding 500-750 µL solution M₆ to the mixture,
- Centrifuging the mixture at 24-26°C for 1-2 minutes at the rate of 6000-11000xg and discharging the bottom chamber,
- Centrifuging the DNA bonding column (1) at 24-26°C for 1-2 minutes at the rate of 6000-11000xg,
- Separating the DNA bonding column (1) from the exterior chamber and transferring into the tube,
- Adding 30-200 µL solution M₇ kept at 50-70°C to the center of the silica column,
- Incubating the mixture at 50-70°C for 5-10 minutes, and
- Centrifuging the mixture at 24-26°C for 1-2 minutes at the rate of 6000-11000xg.

In the method of the invention, the incubation periods of solutions M₁ and M₂ can be prolonged for DNA isolation depending on the species and tissues. The incubation period of solution M₁ at the tissue fractionation and cell lesion phase can be in the range of 10-30 minutes, and the incubation period of solutions M_{2A} and M_{2B} at the expositing the DNA molecules phase can be in the range of 20-40 minutes.

The volume of solution M₇ can be reduced down to 0,03 mL at the phase of washing and separation of the DNA molecule from silica at the DNA isolation kit in cases where it is required to extract 0,5-1,0 micrograms of DNA per microliter.

The temperature can be increased up to 70-85°C in order to achieve effective decomposition for plant species such as Origanum, Thymus, hemp, cumin, etc. at the phase of washing and separation of the DNA molecule from silica.

It is possible to add chloroform at identical stoichiometric ratios (1/1) by volume after addition of solution M₂ at the phase of expositing the DNA molecules for the tissues such as pericarp, petal, placenta, sepal, etc. that are rich in pigment and polysaccharides. Furthermore, for these tissues, the phase expositing the DNA molecules comprises the following process steps after the centrifuging process;
- Adding 2,4-5,6 M NaCl at stoichiometric ratio of 0,1 (1/0,1) by volume and isopropanol at stoichiometric ratio of 0,9 (1/0,9) by volume on 0,5 mL supernatant,
- Centrifuging the solution at 24-26°C for 10 minutes at the rate of 6000-11000xg, and
- Dissolving the pellets obtained in 0,25-0,39 mL solution M₇. After these process steps, the phase of bonding of the DNA molecules to the silica particles can be maintained without any changes.

DNA isolation has been performed at the DNA bonding columns (1) using the DNA solutions especially from seed, radicle and pedicle parts of the members of *Poaceae* family such as wheat, barley, oat, paddy, triticale, corn, stolon agrostis tenuis; from ovule, seed, micro tuber, pedicle, root, leaf, pericarp, placenta, tuber, and stolon parts of the member of *Solanaceae* family, such as aubergine, potato, tomato, pepper; from leave, ovule, seed, root, sepal, petal, pollen parts of the cotton species (*Gossypium hirsutum L.* and *G*. *barbadense L.*); from the seeds of the member of *Leguminosae* family, such as lentil, beans, pea, trefoil and the leaves of medical aromatic plants, such as laurel, raziyane, sage, thyme etc. The quality and quantity of the isolated DNA samples have been determined through spectrophotometric measurements. In addition, the samples have been subjected to agarose gel electrophoresis technique and determined using ethidium bromide dyeing technique.

### Form of Implementing the Invention in the Industry:

The genomic DNA isolation method of the invention, that is economic, high quality and efficient, free of carcinogenic and mutagenic agents, with no negative impact on the environment and the researcher (applicator) in terms of health, and that can be used effectively on generative and vegetative plant parts and many plant varieties and species, can be qualified as having superior characteristics than other DNA isolation methods.

## Claims

1. A genomic DNA isolation method, **characterized in that** the DNA isolation method comprises the process steps of;
**A. Tissue fractionation, Expositing the cells and cell lesion,**
• Fractionating the naturally dried or artificially dried plant parts, plant seeds and lyophilized samples at micro mills, and the fresh plant parts in liquid nitrogen,
• Collecting 0,03-0,08 g samples from dried plant parts, and 0,1-0,3 g samples from fresh tissue parts, and 0,1-0,15 g from plant seeds and placing the samples into 1,5-2,0 mL tubes,
• Adding 300-600 µL M₁ solution and 10-20 µL 20 mg/mL Proteinase K enzyme to the plant samples wherein the M₁ solution is prepared by the process steps of;
- Mixing 1-3 M Tris (pH 7,5-8,5) and 0,5-1,5 M (pH 7,5-8,5) EDTA,
- Adding Tris-EDTA mixture to distilled water,
- Autoclaving and sterilizing the mixture,
- Adding 12,5-25,5 mL 10-15% SDS, 12,5-25,5 mL 10-15% SLS, 12,5-15,5 mL 5-10% SDC, 25-50 mL 5-10% PVPP, 2,5-5,5 mL Triton-X-100, 2,5-5,5 mL Tween-20 and 1,2-2,75 mL 4-8% NaN3 to the mixture,
- Filtering and sterilizing the solution,
• Mixing the obtained solutions for 30-90 seconds,
• Keeping the mixtures at 50-70°C for 10-20 minutes and agitating the mixtures for 30-45 seconds once per every 5 minutes,
**B. Expositing the DNA molecules,**
• Adding solution M_{2A} or M_{2B} to the mixtures at half the stoichiometric ratio (1/2) of solution M₁ by volume, wherein the solution M_{2A} is prepared by the process steps of;
- Dissolving 67,8-78,8 g potassium acetate (KCH3COOH) in 60-80 mL distilled water,
- Adding 23-30 mL 12-14 M glacial acetic acid to the solution,
- Agitating the mixture,
- Adding 65-85 mL distilled water to the mixture,
and wherein the solution M_{2B} is prepared by the process steps of;
- Mixing 42,8-56,2 g guanidine HCl and 30-42 mL 3,4-4,8 M NaCl,
- Adding distilled water until the volume of the solution reaches 100 mL,
• Mixing the obtained solutions for 30-60 seconds,
• Keeping the mixtures at 50-70°C for 10-20 minutes and agitating the mixtures for 15-45 seconds once per every 5 minutes,
• Following incubation, centrifuging the solution at 24-26°C for 10-20 minutes at the rate of 6000-12000xg,
**C. Bonding of the DNA molecules to the silica particles,**
• Adding 0,25-0,5 mL solution M₃ and 0,25-0,5 mL ethanol or isopropanol to supernatant, wherein the solution M₃ is prepared by the process steps of;
- Adding 50-80 mL M2 solution to 105-115 g guanidine HCl,
- Adding 12,5-25,5 mL 80-100% Triton-X-100 and 67,5-87,5 mL distilled water to the mixture
• Agitating the mixtures for 30-60 seconds,
• Transferring 0,5-0,7 mL to a DNA bonding column (1),
• Centrifuging said DNA bonding column (1) at 24-26°C for 1-2 minutes at the rate of 6000-11000xg and decanting the solution collected at the exterior tube,
• Repeating transfer of 0,5-0,7 mL solution to said DNA bonding column (1) and bonding of the DNA molecules to the silica particles,
• Adding 0,25-0,5 mL M₄ solution to the inner chamber of said DNA bonding column (1) for the purpose of selective bonding of the DNA molecules to the silica particles and removal of the pigments, wherein the M₄ solution is prepared by the process steps of;
- Adding 165-195 mL 96-100% ethyl alcohol and 12,5-25,5 mL 80-100% Triton-X-100 to 37,8-54,3 g guanidine HCl,
- Adding ethyl alcohol to the mixture in order to increase the volume of the mixture to 200-250 mL, and
• Centrifuging the mixture at 24-26°C for 1-2 minutes at the rate of 6000-11000xg and decanting the solution collected at the exterior tube,
**D. Washing and separation of the DNA molecule from silica,**
• Adding 500-750 µL solution M₅ to said DNA bonding column (1), wherein the solution M₅ is prepared by the process steps of;
- Mixing 38,75-49,75 mL water with 25 mL 0,5-1,5 M MOPS (pH 7,2-7,9),
- Adding 50-75 mL 3,6-4,4 M NaCl and 1,25-2,50 mL 2-4% (w/v) NaN3 to the mixture,
- Adding 25-50 mL M2A solution and 50-100 mL 96-100% ethyl alcohol to the mixture,
• Centrifuging the mixture at 24-26°C for 1-2 minutes at the rate of 6000-11000xg and discharging the bottom chamber,
• Adding 500-750 µL solution M₆ to the mixture, wherein the solution M₆ is prepared by the process steps of;
- Mixing 8-12 mM Tris and 0,5-1,2 mM EDTA (pH 7,4-8,4),
- Adding 0,08-1,25 mL 5 M NaCl and 0,25-0,50 mL of 4-8% NaN3 to the mixture,
- Adding 5,67-14,6 mL distilled water and 175-195 mL 96-100% ethyl alcohol to the mixture,
• Centrifuging the mixture at 24-26°C for 1-2 minutes at the rate of 6000-11000xg and discharging the bottom chamber,
• Centrifuging said DNA bonding column (1) at 24-26°C for 1-2 minutes at the rate of 6000-11000xg,
• Separating said DNA bonding column (1) from the exterior chamber and transferring into the tube,
• Adding 30-200 µL solution M₇ kept at 50-70°C to the center of the silica column, wherein the solution M₇ is prepared by the process steps of;
- Mixing 25-35 mL 0,01-0,06 M Tris (pH 7,5-8,5) with 175-225 mL distilled water,
• Incubating the mixture at 50-70°C for 5-10 minutes, and
• Centrifuging the mixture at 24-26°C for 1-2 minutes at the rate of 6000-11000xg.

2. A DNA isolation method according to Claim 1, **characterized in that** the method for obtaining 1^{st} DNA bonding column (1) comprises the process steps of;
• Placing the Filter papers within the tube using a sterile forceps and loading bar and adding 15-67 mg silica thereon,
• After the silica surface equalizes, pressing the O-ring on at least one filter paper using the loading bar,
• Obtaining column equilibrium solution (CES) by mixing 3 mL 0,2-1,2 M 3-morpholino propane-1-sulfonic acid (MOPS, pH 6,5-7,5), 4,5 mL 2,4-5,6 M sodium chloride (NaCl), 17,55-21,55 mL distilled water, 0,45-0,65 mL Triton-X-100 and 2,5-6,5 mL isopropanol in a separate tube,
• Adding 0,20-0,75 mL column equilibrium solution (CES) to said column (1),
• Keeping the mixture at 22-26°C for 1-3 hours, and,
• Centrifuging the mixture for 1-2 minutes at the rate of 6000-12000xg.

3. A DNA isolation method according to Claim 1, **characterized in that**, the method for obtaining 2^{nd} DNA bonding column (1) from used DNA bonding column (1) comprises the process steps of;
• Sorting the used DNA bonding column (1) according to the type of components such as exterior tube, inner tube, silica, supporting layers and O-ring,
• Keeping the components within 0,01-0,7 N hydrochloric acid (HCl) at stoichiometric proportion of at least 3 times by volume (3/1) for 25-36 hours,
• Rinsing the components with distilled water for at least 3 times and autoclaving,
• Adding, consecutively for 2-5 times, 0,5-0,75 mL 0,01-0,7 N HCl to the columns (1),
• Centrifuging the mixture for 1-2 minutes at the rate of 6000-12000xg,
• Centrifuging empty DNA bonding column (1) for 1-2 minutes at the rate of 6000-12000xg,
• Adding 0,5-0,75 mL CES to the columns (1) and keeping the same at 22-26°C for 1-3 hours, and
• Centrifuging the mixture for 1-2 minutes at the rate of 6000-12000xg.

4. A DNA isolation method according to Claim 1, **characterized in that**, the volume of solution M₇ can be reduced down to 0,03 mL at the phase of washing and separation of the DNA molecule from silica in cases where it is required to extract 0,5-1,0 micrograms of DNA per microliter.

5. A DNA isolation method according to Claim 1, **characterized in that**, the temperature can be increased up to 70-85°C in order to achieve effective decomposition for plant species such as Origanum, Thymus, hemp and cumin at the phase of washing and separation of the DNA molecule from silica.

6. A DNA isolation method according to Claim 1, **characterized in that**, at the phase of expositing the DNA molecules, the expositing the DNA molecules comprises the following process steps after the centrifuging process;
• Adding 2,4-5,6 M NaCl at stoichiometric ratio of 0,1 (1/0,1) by volume and isopropanol at stoichiometric ratio of 0,9 (1/0,9) by volume on 0,5 mL supernatant,
• Centrifuging the solution at 24-26°C for 10 minutes at the rate of 6000-11000xg, and
• Dissolving the pellets obtained in 0,25-0,39 mL solution M₇.

## Patentansprüche

1. Genomische DNA-Isolierungsverfahren, **dadurch gekennzeichnet, dass**, das DNA-Isolierungsverfahren die folgenden Verfahrensschritte umfasst;
**A. Fraktionierung des Gewebes, Freilegung der Zellen und Zellläsion,**
• Fraktionierung der natürlich getrockneten oder künstlich getrockneten Pflanzenteile, Pflanzensamen und lyophilisierten Proben in Mikromühlen und der frischen Pflanzenteile in flüssigem Stickstoff,
• Sammlung von 0,03-0,08 g Proben von getrockneten Pflanzenteilen, und von 0,1-0,3 g Proben von frischen Gewebeteilen und 0,1-0,15 g von Pflanzensamen und Platzierung der Proben in 1,5-2,0 mL Rohren,
• Zugabe von 300-600 µL M₁-Lösung und 10-20 µL 20 mg/mL Proteinase K-Enzyme zu den Pflanzenproben, wobei die M₁-Lösung durch die folgenden Verfahrensschritte hergestellt wird;
- Mischen von 1-3 M Tris (pH 7,5-8,5) und 0,5-1,5 M (pH 7,5-8,5) EDTA,
- Zugabe von Tris-EDTA-Mischung zu destilliertem Wasser,
- Autoklavieren und Sterilisieren der Mischung,
- Zugabe von 12,5-25,5 mL 10-15% SDS, 12,5-25,5 mL 10-15% SLS, 12,5-15,5 mL 5-10% SDC, 25-50 mL 5-10% PVPP, 2,5-5,5 mL Triton-X-100, 2,5-5,5 mL Tween-20 und 1,2-2,75 mL 4-8% NaN3 zu der Mischung,
- Filtrieren und Sterilisieren der Lösung,
• Mischen der erhaltenen Lösungen für 30-90 Sekunden,
• Halten der Mischungen bei 50-70°C für 10-20 Minuten und Schütteln der Mischungen für 30-45 Sekunden ein Mal pro 5 Minuten,
**B. Freilegung der DNA-Moleküle,**
• Zugabe von M_{2A}-Lösung oder M_{2B}-Lösung zu den Mischungen im halben stöchiometrischen Verhältnis (1/2) von M₁-Lösung in Volumensanteil, wobei die M_{2A}-Lösung durch die folgenden Verfahrensschritte hergestellt wird;
- Auflösen von 67,8-78,8 g Kaliumacetat (KCH3COOH) in 60-80 mL destilliertem Wasser,
- Zugabe von 23-30 mL 12-14 M Eisessigsäure zu der Lösung,
- Schütteln der Mischung,
- Zugabe von 65-85 mL destilliertem Wasser zu der Mischung,
und wobei die M_{2B}-Lösung durch die folgenden Verfahrensschritte hergestellt wird;
- Mischen von 42,8-56,2 g Guanidin HCl und 30-42 mL 3,4-4,8 M NaCl,
- Zugabe von destilliertem Wasser, bis das Volumen der Lösung 100 mL erreicht,
• Mischen der erhaltenen Lösungen für 30-60 Sekunden,
• Halten der Mischungen bei 50-70°C für 10-20 Minuten und Schütteln der Mischungen für 15-45 Sekunden ein Mal pro 5 Minuten,
• Im Anschluss an die Inkubation wird die Lösung bei 24-26°C für 10-20 Minuten bei einer Geschwindigkeit von 6000-12000xg zentrifugiert,
**C. Bindung der DNA-Moleküle an die Silikapartikel,**
• Zugabe von 0,25-0,5 ml M₃-Lösung und 0,25-0,5 ml Ethanol oder Izopropanol zum Überstand, wobei die M₃-Lösung durch die folgenden Verfahrensschritte hergestellt wird;
- Zugabe von 50-80 mL M2-Lösung zu 105-115 Guanidin-HCl,
- Zugabe von 12,5-25,5 mL 80-100% Triton-X-100 und 67,5-87,5 mL destilliertem Wasser zu der Mischung
• Schütteln der Mischungen für 30-60 Sekunden,
• Übertragen von 0,5-0,7 mL auf eine DNA-Bindungssäule (1),
• Zentrifugieren der genannten DNA-Bindungssäule (1) bei 24-26°C für 1-2 Minuten bei einer Geschwindigkeit von 6000-11000xg und Dekantieren der am äußeren Rohr gesammelten Lösung,
• Wiederholte Übertragung von 0,5-0,7 mL Lösung auf die genannte DNA-Bindungssäule (1) und Bindung der DNA-Moleküle an die Silikapartikel,
• Zugabe von 0,25-0,5 mL M₄-Lösung in die innere Kammer der genannten DNA-Bindungssäule (1) zum Zweck der selektiven Bindung der DNA-Moleküle an die Silikapartikel und Entfernung der Pigmente, wobei die M₄-Lösung durch die folgenden Verfahrensschritte hergestellt wird;
- Zugabe von 165-195 mL 96-100% Ethylalkohol und 12,5-25,5 mL 80-100% Triton-X-100 zu 37,8-54,3 g Guanidin HCl,
- Zugabe von Ethylalkohol zu der Mischung, um das Volumen der Mischung auf 200-250 mL zu erhöhen, und
• Zentrifugieren der Mischung bei 24-26°C für 1-2 Minuten bei einer Geschwindigkeit von 6000-11000xg und Dekantieren der am äußeren Rohr gesammelten Lösung,
**D. Waschen und Trennen des DNA-Moleküls vom Silika,**
• Zugabe von 500-750 µL M₅-Lösung zu der genannten DNA-Bindungssäule (1), wobei die M₅-Lösung durch die folgenden Verfahrensschritte hergestellt wird;
- Mischen von 38,75-49,75 mL Wasser mit 25 mL 0,5-1,5 M MOPS (pH 7,2-7,9),
- Zugabe von 50-75 mL 3,6-4,4 M NaCl und 1,25-2,50 mL 2-4% (w/v) NaN3 zu der Mischung,
- Zugabe von 25-50 mL M_{2A} Lösung und 50-100 mL 96-100% Ethylalkohol zu der Mischung,
• Zentrifugieren der Mischung bei 24-26°C für 1-2 Minuten bei einer Geschwindigkeit von 6000-11000xg und Entleerung der unteren Kammer,
• Zugabe von 500-750 µL M₆-Lösung zu der Mischung, wobei die M₆-Lösung durch die folgenden Verfahrensschritte hergestellt wird;
- Mischen von 8-12 mM Tris und 0,5-1,2 mM EDTA (pH 7,4-8,4),
- Zugabe von 0,08-1,25 mL 5 M NaCl und 0,25-0,50 mL von 4-8% NaN3 zu der Mischung,
- Zugabe von 5,67-14,6 mL destilliertem Wasser und 175-195 mL 96-100% Ethylalkohol zu der Mischung,
• Zentrifugieren der Mischung bei 24-26°C für 1-2 Minuten bei einer Geschwindigkeit von 6000-11000xg und Entleerung der unteren Kammer,
• Zentrifugieren der genannten DNA-Bindungssäule (1) bei 24-26°C für 1-2 Minuten bei einer Geschwindigkeit von 6000-11000xg,
• Trennen der genannten DNA-Bindungssäule (1) von der äußeren Kammer und Übertragen in das Rohr,
• Zugabe von 30-200 µL M₇-Lösung, die auf 50-70°C gehalten wird, in die Mitte der Silikasäule, wobei die M₇-Lösung durch die folgenden Verfahrensschritte hergestellt wird;
- Mischen von 25-35 mL 0,01-0,06 M Tris (pH 7,5-8,5) mit 175-225 mL destilliertem Wasser,
• Inkubation der Mischung bei 50-70°C für 5-10 Minuten, und
• Zentrifugieren der Mischung bei 24-26°C für 1-2 Minuten bei einer Geschwindigkeit von 6000-11000xg.

2. DNA-Isolierungsverfahren nach Anspruch 1, **dadurch gekennzeichnet, dass**, das Verfahren zur Erhaltung der 1. DNA-Bindungssäule (1) die folgenden Verfahrensschritte umfasst;
• Platzieren der Filterpapiere im Rohr unter Verwendung einer sterilen Pinzette und eines Ladestabes und Zugabe von 15-67 mg Silika darauf,
• Nach dem Abgleich der Silika-Oberfläche wird der O-Ring unter Verwendung des Ladestabes auf mindestens ein Filterpapier gedrückt,
• Erhalten einer Säulen-Gleichgewichtslösung (CES) durch Mischen von 3 mL 0,2-1,2 M 3- Morpholino-propan-1-Sulfonsäure (MOPS, pH 6,5-7,5), 4,5 mL 2,4-5,6 M Natriumchlorid (NaCl), 17,55-21,55 mL destilliertem Wasser, 0,45-0,65 mL Triton-X-100 und 2,5-6,5 mL Izopropanol in einem separaten Rohr,
• Zugabe von 0,20-0,75 mL Säulen-Gleichgewichtslösung (CES) zu der genannten Säule (1),
• Halten die Mischung bei 22-26°C für 1-3 Stunden, und,
• Zentrifugieren der Mischung für 1-2 Minuten bei einer Geschwindigkeit von 6000-12000xg.

3. DNA-Isolierungsverfahren nach Anspruch 1, **dadurch gekennzeichnet, dass**, das Verfahren zur Erhaltung der 2. DNA-Bindungssäule (1) aus der gebrauchten DNA-Bindungssäule (1) umfasst die folgenden Verfahrensschritte;
• Sortieren der gebrauchten DNA-Bindungssäule (1) nach der Art der Komponenten wie Außenrohr, Innenrohr, Silika, Stützschichten und O-Ring,
• Halten der Komponenten in 0,01-0,7 N Salzsäure (HCl) bei einem stöchiometrischen Verhältnis von mindestens dem 3-mal Volumensanteil (3/1) für 25-36 Stunden,
• Spülen der Komponenten mit destilliertem Wasser für mindestens 3-mal und Autoklavieren,
• Zugabe von 2-5-mal nacheinander 0,5-0,75 mL 0,01-0,7 N HCl zu den Säulen (1),
• Zentrifugieren der Mischung für 1-2 Minuten bei einer Geschwindigkeit von 6000-12000xg,
• Zentrifugieren der leeren DNA-Bindungssäule (1) für 1-2 Minuten bei einer Geschwindigkeit von 6000-12000xg,
• Zugabe von 0,5-0,75 mL CES zu den Säulen (1) und Halten derselben bei 22-26°C für 1-3 Stunden, und
• Zentrifugieren der Mischung für 1-2 Minuten bei einer Geschwindigkeit von 6000-12000xg.

4. DNA-Isolierungsverfahren nach Anspruch 1, **dadurch gekennzeichnet, dass**, das Volumen der M₇-Lösung kann in der Phase des Waschens und der Trennung des DNA-Moleküles vom Silika auf 0,03 ml reduziert werden, falls eine Extraktion von 0,5-1,0 Mikrogramm DNA pro Mikroliter erforderlich ist.

5. DNA-Isolierungsverfahren nach Anspruch 1, **dadurch gekennzeichnet, dass**, die Temperatur kann bis auf 70-85°C erhöht werden, um einen effektiven Abbau für Pflanzengattungen wie Origanum, Thymus, Hanf und Kümmel in der Phase des Waschens und der Trennung des DNA-Moleküles von der Silika zu erreichen.

6. DNA-Isolierungsverfahren nach Anspruch 1, **dadurch gekennzeichnet, dass**, in der Phase des Freilegens der DNA-Moleküle umfasst das Freilegen der DNA-Moleküle die folgenden Verfahrensschritte nach dem Zentrifugierprozess;
• Zugabe von 2,4-5,6 M NaCl im stöchiometrischen Verhältnis von 0,1 (1/0,1) Volumenanteilen und Izopropanol im stöchiometrischen Verhältnis von 0,9 (1/0,9) Volumenanteilen auf 0,5 mL Überstand,
• Zentrifugieren der Lösung bei 24-26°C für 10 Minuten bei einer Geschwindigkeit von 6000-11000xg, und
• Auflösen der erhaltenen Pellets in 0,25-0,39 mL M₇-Lösung.

## Revendications

1. Procédé d'isolement d'ADN génomique, **caractérisé en ce que** le procédé d'isolement d'ADN comprend les étapes de procédé qui consiste à:
**A. Le fractionnement des tissus, l'exposition des cellules et la lésion cellulaire,**
• Fractionner les parties de plantes séchées de manière naturelle ou artificielle, les graines de plantes et les échantillons lyophilisés dans des micro-broyeurs, et les parties de plantes fraîches dans de l'azote liquide,
• Prélever des échantillons de 0,03-0,08 g sur des parties de plantes séchées, et des échantillons de 0,1-0,3 g sur des parties de tissus frais, et 0,1-0,15 g de graines de plantes et placer les échantillons dans des tubes de 1,5 -2,0 mL,
• Ajouter 300 à 600 µL de solution M1 et 10 à 20 µL d'enzyme Protéinase K à 20 mg/mL aux échantillons de plantes dans lesquels la solution M₁ est préparée par les étapes de procédé de;
- Mélange de 1-3 M Tris (pH 7,5-8,5) et 0,5-1,5 M (pH 7,5-8,5) EDTA,
- Ajout du mélange Tris-EDTA à de l'eau distillée,
- Autoclaver et stériliser le mélange,
- Ajout de 12,5-25,5 mL 10-15% SDS, 12,5-25,5 mL 10-15% SLS, 12,5-15,5 mL 5-10% SDC, 25-50 mL 5-10 % PVPP, 2,5-5,5 mL Triton-X-100, 2,5-5,5 mL Tween-20 et 1,2-2,75 mL 4-8% NaN3 au mélange,
- Filtration et stérilisation de la solution,
• Mélange des solutions obtenues pendant 30-90 secondes,
• Conservation des mélanges à 50-70°C pendant 10-20 minutes et agiter les mélanges pendant 30 à 45 secondes une fois toutes les 5 minutes,
**B. Exposition des molécules d'ADN,**
• Ajout de la solution M_{2A} ou M_{2B} aux mélanges à la moitié du ratio stœchiométrique (1/2) de la solution M₁ en volume, dans laquelle la solution M_{2A} est préparée par les étapes de procédé de;
- Dissolution de 67,8-78,8 g d'acétate de potassium (KCH3COOH) dans 60-80 mL d'eau distillée,
- Ajout de 23-30 ml d'acide acétique glacial 12-14 M à la solution,
- Agitation du mélange,
- Ajout de 65-85 ml d'eau distillée au mélange,
et dans lequel la solution M_{2B} est préparée par les étapes de procédé de;
- Mélange de 42,8-56,2 g de guanidine HCl et 30-42 mL 3,4-4,8 M NaCl,
- Ajout de l'eau distillée jusqu'à ce que le volume de la solution atteigne 100 ml,
• Mélange des solutions obtenues pendant 30-60 secondes,
• Conservation des mélanges à 50-70°C pendant 10-20 minutes et agiter les mélanges pendant 15-45 secondes une fois toutes les 5 minutes,
• Suite à l'incubation, centrifugation de la solution à 24-26°C pendant 10-20 minutes à raison de 6000-12000xg,
**C. Liaison des molécules d'ADN aux particules de silice,**
• Ajout de 0,25-0,5 ml de solution M₃ et 0,25-0,5 mL d'éthanol ou d'isopropanol au surnageant, dans lequel la solution M₃ est préparée par les étapes de procédé de;
- Ajout de 50-80 mL de solution M₂ à 105 à 115 g de guanidine HCl,
- Ajout de 12,5-25,5 mL 80-100% Triton-X-100 et 67,5-87,5 mL d'eau distillée au mélange
• Agitation des mélanges pendant 30-60 secondes,
• Transfert de 0,5-0,7 mL sur une colonne de liaison à l'ADN (1),
• Centrifugation de ladite colonne de liaison à l'ADN (1) à 24-26°C pendant 1-2 minutes à raison de 6000-11000xg et décantation de la solution collectée au niveau du tube extérieur,
• Répétition du transfert de 0,5-0,7 mL de solution à ladite colonne de liaison à l'ADN (1) et la liaison des molécules d'ADN aux particules de silice,
• Ajout de 0,25 à 0,5 ml de solution M₄ à la chambre intérieure de ladite colonne de liaison à l'ADN (1) dans le but d'une liaison sélective des molécules d'ADN aux particules de silice et de l'élimination des pigments, la solution M₄ étant préparée en les étapes du procédé de;
- Ajout de 165-195 mL 96-100% d'alcool éthylique et 12,5-25,5 mL 80-100% Triton-X-100 à 37,8-54,3 g de guanidine HCl,
- Ajout de l'alcool éthylique au mélange afin d'augmenter le volume du mélange à 200-250 mL, et
• Centrifugation du mélange à 24-26°C pendant 1-2 minutes à raison de 6000-11000xg et décantation de la solution collectée au niveau du tube extérieur,
**D. Lavage et séparation de la molécule d'ADN de la silice,**
• Ajout de 500 à 750 µL de solution M₅ à ladite colonne de liaison à l'ADN (1), dans laquelle la solution M₅ est préparée par les étapes de procédé de;
- Mélange de 38,75-49,75 ml d'eau avec 25 ml de MOPS 0,5-1,5 M (pH 7,2-7,9),
- Ajout de 50-75 ml 3,6-4,4 M NaCl et 1,25-2,50 ml 2-4% (w/v) NaN3 au mélange,
- Ajout de 25-50 ml de solution M_{2A} et 50-100 ml d'alcool éthylique à 96-100% au mélange,
• Centrifugation du mélange à 24-26°C pendant 1-2 minutes à raison de 6000-11000xg et décharge de la chambre inférieure,
• Ajout de 500-750 µL de solution M₆ au mélange, dans laquelle la solution M₆ est préparée par les étapes de procédé de;
- Mélange de 8-12 mM Tris et 0,5-1,2 mM EDTA (pH 7,4-8,4),
- Ajout de 0,08-1,25 mL de NaCl 5 M et 0,25-0,50 mL de 4-8% NaN3 au mélange,
- Ajout de 5,67-14,6 mL d'eau distillée et 175-195 mL 96-100% d'alcool éthylique au mélange,
• Centrifugation du mélange à 24-26°C pendant 1-2 minutes à raison de 6000-11000xg et décharge de la chambre inférieure,
• Centrifugation de ladite colonne de liaison à l'ADN (1) à 24-26°C pendant 1-2 minutes à raison de 6000-11000xg,
• Séparation de ladite colonne de liaison à l'ADN (1) de la chambre extérieure et transfert dans le tube,
• Ajout de 30-200 µL de solution M₇ conservée à 50-70°C au centre de la colonne de silice, dans laquelle la solution M₇ est préparée par les étapes de procédé de;
- Mélange de 25-35 mL 0,01-0,06 M Tris (pH 7,5-8,5) avec 175-225 mL d'eau distillée,
• Incubation du mélange à 50-70°C pendant 5-10 minutes, et
• Centrifugation du mélange à 24-26°C pendant 1-2 minutes à raison de 6000-11000xg.

2. Procédé d'isolement d'ADN selon la revendication 1, **caractérisé en ce que** le procédé d'obtention de la 1^{ère} colonne de liaison d'ADN (1) comprend les étapes de procédé consistant à:
• Placer les papiers Filtres dans le tube à l'aide d'une pince stérile et d'une barre de chargement et ajouter 15 à 67 mg de silice dessus,
• Une fois la surface de silice égalisée, presser le joint torique sur au moins un papier filtre à l'aide de la barre de chargement,
• Obtenir une solution d'équilibre de colonne (CES) en mélangeant 3 mL d'acide 0,2-1,2 M 3-morpholino propane-1-sulfonique (MOPS, pH 6,5-7,5), 4,5 mL 2,4-5 ,6 M de chlorure de sodium (NaCl), 17,55-21,55 ml d'eau distillée, 0,45-0,65 ml de Triton-X-100 et 2,5-6,5 ml d'isopropanol dans un tube séparé,
• Ajouter 0,20-0,75 ml de solution d'équilibre de colonne (CES) à ladite colonne (1),
• Conserver le mélange à 22-26°C pendant 1-3 heures, et,
• Centrifuger le mélange pendant 1-2 minutes à raison de 6000-12000xg.

3. Procédé d'isolement d'ADN selon la revendication 1, **caractérisé en ce que** le procédé d'obtention d'une 2^{ème} colonne de liaison d'ADN (1) à partir d'une colonne de liaison d'ADN utilisée (1) comprend les étapes de procédé consistant à:
• Trier la colonne de liaison ADN utilisée (1) selon le type de composants tels que tube extérieur, tube intérieur, silice, couches de support et joint torique,
• Maintenir les composants dans l'acide chlorhydrique 0,01-0,7 N (HCl) à une proportion stœchiométrique d'au moins 3 fois en volume (3/1) pendant 25-36 heures,
• Rincer les composants à l'eau distillée au moins 3 fois et autoclavage,
• Ajouter, consécutivement 2 à 5 fois, 0,5-0,75 mL 0,01-0,7 N HCl aux colonnes (1),
• Centrifuger le mélange pendant 1-2 minutes à raison de 6000-12000xg,
• Centrifuger la colonne de liaison à l'ADN vide (1) pendant 1-2 minutes à raison de 6000-12000xg,
• Ajouter 0,5-0,75 ml de CES aux colonnes (1) et les maintenir à 22-26°C pendant 1-3 heures, et
• Centrifuger le mélange pendant 1-2 minutes à raison de 6000-12000xg.

4. Procédé d'isolement d'ADN selon la revendication 1, **caractérisé en ce que** le volume de solution M₇ peut être réduit jusqu'à 0,03 mL lors de la phase de lavage et de séparation de la molécule d'ADN de la silice lorsqu'il est nécessaire d'en extraire 0, 5-1,0 microgrammes d'ADN par microlitre.

5. Procédé d'isolement d'ADN selon la revendication 1, **caractérisé en ce que** la température peut être élevée jusqu'à 70-85°C pour d'obtenir une décomposition efficace pour les espèces végétales telles que l'Origan, le Thymus, le chanvre et le cumin lors de la phase de lavage et de séparation de la molécule d'ADN de silice.

6. Procédé d'isolement d'ADN selon la revendication 1, **caractérisé en ce que**, à la phase d'exposition des molécules d'ADN, l'exposition des molécules d'ADN comprend les étapes de procédé suivantes après le procédé de centrifugation;
• Ajout de NaCl 2,4-5,6 M à un ratio stœchiométrique de 0,1 (1/0,1) en volume et d'isopropanol à un ratio stœchiométrique de 0,9 (1/0,9) en volume sur 0,5 mL de surnageant,
• Centrifugation de la solution à 24-26°C pendant 10 minutes à raison de 6000-11000xg, et
• Dissolution des pellets obtenus dans 0,25-0,39 mL de solution M₇.
